# EUROPEAN PATENT SPECIFICATION

(11) **EP 2 152 291 B1**
(45) Date of publication and mention of the grant of the patent: **24.10.2012**
(21) Application number: 08747428.4
(22) Date of filing: 02.05.2008
(51) Int. Cl.: A61P 27/00, A61K 38/13, A61F 2/16

(54) **USE OF CYCLOSPORINES IN THE TREATMENT OF PATIENTS WITH INTRAOCULAR LENSES**
VERWENDUNG VON CYCLOSPORINEN BEI DER BEHANDLUNG VON PATIENTEN MIT INTRAOKULARLINSEN
UTILISATION DE CYCLOSPORINES DANS LE TRAITEMENT DE PATIENTS PORTEURS DE LENTILLES INTRA-OCULAIRES

(30) Priority: 04.05.2007 US 915950 P
(43) Date of publication of application: 17.02.2010
(73) Proprietor: ALLERGAN, INC., Irvine, CA 92612 (US)
(72) Inventor: DONNENFELD, Eric, D., Rockville Centre, NY 11570 (US)
(74) Representative: HOFFMANN EITLE
(86) International application number: PCT/US2008/062325
(87) International publication number: WO 2008/137617

(56) References cited:
- WO-A-2006/014738
- US-A- 4 649 047
- US-A1- 2005 063 996
- KADAYIFÇILAR SIBEL ET AL: "Cataract surgery in patients with Behçet's disease." JOURNAL OF CATARACT AND REFRACTIVE SURGERY FEB 2002, vol. 28, no. 2, February 2002 (2002-02), pages 316-320, XP009102263 ISSN: 0886-3350
- DEANGELIS D ET AL: "Cataract extraction by phacoemulsification in a sympathizing eye." CANADIAN JOURNAL OF OPHTHALMOLOGY. JOURNAL CANADIEN D'OPHTALMOLOGIE FEB 2000, vol. 35, no. 1, February 2000 (2000-02), pages 26-28, XP009102287 ISSN: 0008-4182

## Description

This application is based on, and claims the benefit of, U.S. Provisional Application No. 60/915,950, filed May 4, 2007.

The present disclosure relates to methods of treating patients who have received or are to receive an intraocular lens. More particularly, the disclosure relates to methods including administering cyclosporine components to patients, human or animal, who have received or are to receive intraocular lenses.

The use of cyclosporin-A and cyclosporin A derivatives to treat ophthalmic conditions has been the subject of various patents, for example Ding et al U.S. Patent 5,474,979; Garst U.S. Patent 6,254,860; and Garst U.S. 6, 350, 442. In addition, a number of prior art patents have disclosed the use of cyclosporine, administered topically and/or systemically, as a treatment for other conditions and/or diseases.

For example, US 2005/063996 A1 reports intraocular administration of cyclosporin within a lens capsule prior to insertion of a replacement intraocular lens.

US 4,649,047 A reports topical administration of cyclosporin A as an ophthalmic drop or ointment for the treatment of e.g. iatrogenic lens induced uveitis.

WO 2006/014738 A reports the application of cyclosporin to an intraocular lens prior to implanting into the eye.

Kadayifçilar Sibel et al. report administration of systemic cyclosporin A prior to and after IOL implantation (J. Cataract Refract. Surg., 2002, vol. 28, pp. 316-320).

Deangelis D et al. report topical administration of cyclosporin before and/or after IOL implantation at doses of 200, 350, 500 or 150 mg twice daily (Can. J. Ophthalmol., 2000, vol. 35, pp. 26-28).

Intraocular lenses (IOLs) are well known and can be used, when inserted into an eye, for example, on or in the cornea of an eye, in the anterior chamber of an eye, in the posterior chamber of the eye and the like, to correct vision and/or to substitute for the natural lens in the eye which has been removed, for example, because of a cataract condition. In the recent past, multifocal IOLs, that is IOLs with different zones adapted for near and distance or far vision, and accommodating IOLs, that is IOLs which are movable in the eye to provide near and distance or far vision as desired, have been introduced. The IOLs are often inserted into the eye through an ocular surface, for example, through a corneal surface, a scleral surface and the like. An incision, for example, on the order of about 1 mm to about 4 mm or larger may be made.

If necessary, the natural lens is removed, for example, using conventional and well known techniques, such as phacoemulsification and the like. The IOL, often in a folded or rolled configuration, is inserted into the eye through the same incision in the ocular surface. One or more sutures may be used to close the incision. Alternately, the incision is allowed to heal without suturing.

Multifocal IOLs degrade image quality by dividing light energy between a near focus far and a distance or far focus. This can cause a degradation in image quality with a loss of contrast sensitivity. In addition, any irregularity in the ocular surface, for example, in the topography of the ocular surface, may also cause a loss of contrast sensitivity, as well as losses in visual acuity and quality of vision. One or more of these detriments can cause significant patient dissatisfaction with an implanted IOL.

It would be advantageous to mitigate one or more of the above-noted problems which occur with IOL use.

### Summary of the Invention

New methods of treating patients who have received or are to receive intraocular lenses (IOLs) have been discovered. The present methods are relatively straightforward, and can be easily and effectively practiced by the surgeon or prescribing physician and patient without causing substantial or undue patient stress. In addition, the methods advantageously provide one or more benefits to the patient, for example, and without limitation, in terms of quality of vision and ultimately quality of life.

The scope of the present invention is defined by the claims and concerns a cyclosporine component for use in a method of mitigating one or more of the detrimental effects of the insertion of an intraocular lens in an eye by enhancing visual acuity, contrast sensitivity, healing of the ocular surface, quality of vision or reducing wavefront aberrations in the eye of a patient who has received an intraocular lens, the method comprising: topically applying a cyclosporine component to an ocular surface of an eye of a patient, and inserting an intraocular lens in the eye of the patient, wherein the cyclosporine component is applied prior to and after the inserting step.

In one embodiment, the IOL is inserted through the ocular surface of the eye of the patient, and the administering or applying step is effective: (1) to enhance the smoothness of the ocular surface after the inserting step; and/or (2) to enhance the regularity of the topography of the ocular surface after the inserting step; and/or (3) to enhance healing of the ocular surface after the inserting step, each of (1), (2) and (3) being relative to an identical method without the administering or applying step.

The present applying step may be effective: (A) to enhance contrast sensitivity in the eye after the inserting step; and/or (B) to reduce wavefront aberrations in the eye after the inserting step; and/or (C) to enhance visual acuity in the eye after the inserting step; and/or (D) to enhance quality of vision in the eye after the inserting step, each of (A), (B), (C) and (D) being relative to an identical method without the applying step.

Each and every feature described herein, and each and every combination of two or more of such features, is included within the scope of the present invention provided that the features included in such a combination are not mutually inconsistent.

These and other aspects and advantages of the present invention are apparent in the following detailed description, examples and claims.

### Detailed Description

Cyclosporine components are employed to treat patients, humans or animals, who have been or are going to be subjected to surgery to insert intraocular lenses (IOLs).

In general, the methods described herein comprise administering a cyclosporine component to a patient having an eye with an ocular surface; and inserting an IOL in the eye of the patient. The administering step is advantageously effective to mitigate one or more of the detrimental effects of the insertion of the IOL and/or of the presence of the IOL in the eye.

Without wishing to limit the invention to any particular theory of operation, it is believed that the applied cyclosporine component is present in an amount effective in providing or facilitating at least one of the following benefits: smoothing the ocular surface after the inserting step, enhancing healing of the eye after the inserting step, reducing or even substantially eliminating corneal staining after the inserting step, and enhancing visual acuity after the inserting step, each of which benefits is relative to an identical method without the applying step. Moreover, it is believed that with an IOL in place in the eye of the patient, all of the light passing into the eye does not go to one focal point. In this case, the stability, in terms of composition and amount, of the tear film on the eye is important to obtain good, or even substantially optimal results with the IOL for the patient. The question of tear film stability is of particular concern for older patients, that is patients older than about 60 or about 65 or about 70 years of age who are a prime demographic group to have IOLs inserted and who, because of their age, may not have consistently stable tear film.

In one embodiment, the applied cyclosporine component may be effective in promoting or providing a more stable tear film relative to an identical method without the applying step.

The cyclosporine component may be effective in accordance with the present invention: (1) to enhance the smoothness of the ocular surface after the inserting step; and/or (2) to enhance the regularity of the topography of the ocular surface after the inserting step; and/or (3) to enhance healing of the ocular surface after the inserting step. The cyclosporine component may be effective: (A) to enhance contrast sensitivity in the eye after the inserting step; and/or (B) to reduce wavefront aberrations in the eye after the inserting step; and/or (C) to enhance visual acuity in the eye after the inserting step; and/or (D) to enhance quality of vision in the eye after the inserting step, each of (A), (B), (C) and (D) being relative to an identical method without the applying step.

In one useful embodiment, the cyclosporine component is effective to provide or facilitate at least one or (1), (2) and (3); and at least one of (A), (B), (C) and (D).

In any event, the use of the cyclosporine component in combination with inserting an intraocular lens in an eye of a patient in accordance with the present invention is effective and can provide one or more substantial benefits to the patient.

The cyclosporine component is to be applied topically to an eye, for example, an ocular surface of an eye, of a patient. Topical application allows an effective amount of the cyclosporine component to be provided to the eye, substantially without subjecting the remainder of the human or animal to the cyclosporine component.

Employing reduced systemic or blood concentrations of cyclosporine component may also be effective, preferably with substantially no detectable concentration of the cyclosporine component in the blood of the patient being treated. The cyclosporine component concentration of blood can be advantageously measured using a validated liquid chromatography/mass spectrometry-mass spectrometry (VLC/MS-MS) analytical method, which test has a cyclosporine component detection limit of 0.1 ng/ml.

For example the blood of the human or animal patient has concentrations of cyclosporin component of 0.1 ng/ml or less.

The cyclosporine component may be administered to a patient as part of the combination treatment to treat the patient. For example, the cyclosporine component may be administered to the patient along with one or more other therapeutic agents effective in treating the patient. The other therapeutic agent or agents can be administered to the patient in the same composition with the cyclosporine component or in a different composition from the cyclosporine component. Examples of useful other therapeutic components include, without limitation, antibiotics, various pain medications, anti-inflammatory medications and the like and mixtures thereof.

The cyclosporine component may be administered to the patient, prior to, during and after, the surgical procedure or procedures employed to insert the intraocular lens in the eye of the patient. The cyclosporin component is administered at least before and after the intraocular lens inserting step.

Any suitable cyclosporine component effective in the present methods may be used.

Cyclosporines are a group of nonpolar cyclic oligopeptides with known immunosuppressant activity. Cyclosporin A, along with several other minor metabolites, as well as cyclosporin B, C, D, E, F, G, H, I, J, K, L, M, N, O, P, Q, R, S, T, U, V, W, X, Y and Z, have been identified. In addition, derivatives, salts and the like of such cyclosporines and a number of synthetic analogs have been prepared and may be useful in the present invention. See, for example, the Garst Patents noted elsewhere herein.

In general, commercially available cyclosporines may contain a mixture of several individual cyclosporines which all share a cyclic peptide structure consisting of eleven amino acid residues with a total molecular weight of about 1,200, but with different substituents or configurations of some of the amino acids.

The term "cyclosporine component" as used herein is intended to include any individual member of the cyclosporine group, salts thereof, derivatives thereof, analogs thereof and mixtures thereof, as well as mixtures of two or more individual cyclosporines salts thereof, derivatives thereof, analogs thereof and mixtures thereof.

Particularly preferred cyclosporine components include, without limitation, cyclosporin A, derivatives of cyclosporin A, salts of cyclosporin A and the like and mixtures thereof. Cyclosporin A is an especially useful cyclosporine component.

The chemical structure for cyclosporin A is represented by Formula 1

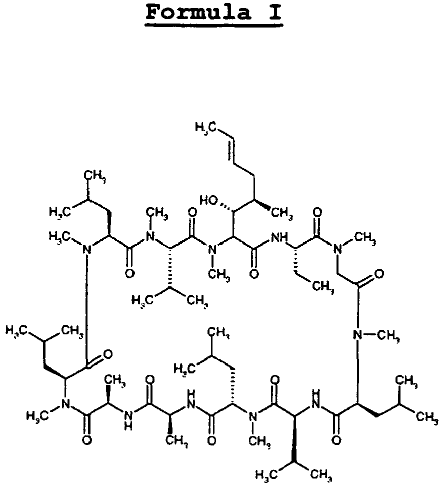

As used herein the term "derivatives" of a cyclosporine refer to compounds having structures sufficiently similar to the cyclosporine so as to function in a manner substantially similar to or substantially identical to the cyclosporine, for example, cyclosporin A, in the present methods. Included, without limitation, within the useful cyclosporin A derivatives are those selected from ((R)-methylthio-Sar)³-(4'-hydroxy-MeLeu) cyclosporin A, ((R)-(Cyclo)alkylthio-Sar)³-(4'-hydroxy-MeLeu)⁴-cyclosporin A, and ((R)-(Cyclo)alkylthio-Sar)³-cyclosporin A derivatives described below.

These cyclosporine derivatives are represented by the following general formulas (II), (III), and (IV) respectively: 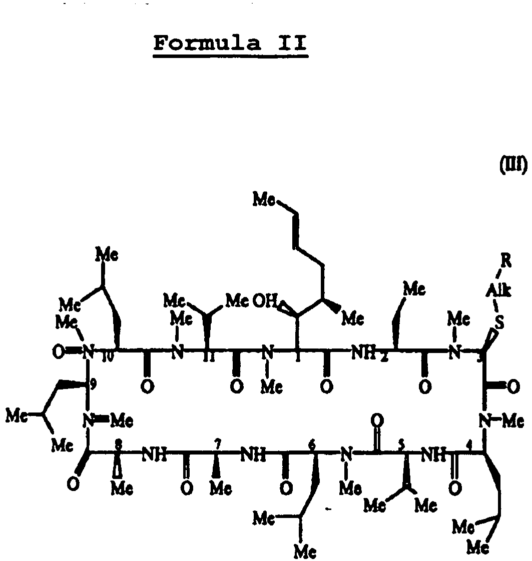 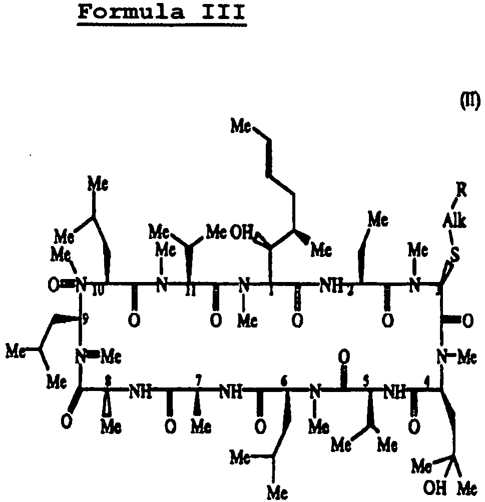 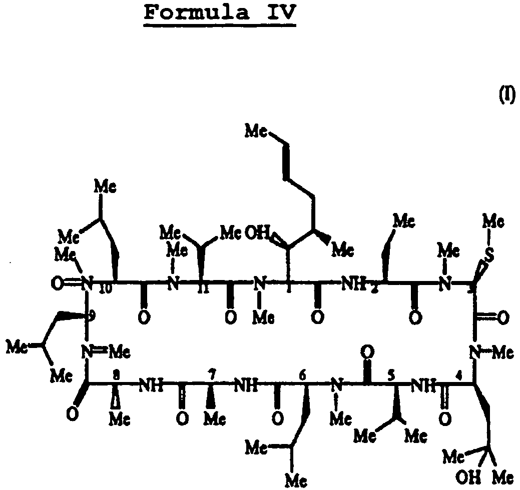 wherein Me is methyl; Alk is 2-6C alkylene or 3-6C cycloalkylene; R is OH, COOH, alkoxycarbonyl, -NR₁R₂ or N(R₃)-(CH₂)-NR₁R₂; wherein R₁, R₂ is H, alkyl, 3-6C cycloalkyl, phenyl (optionally substituted by halo, alkoxy, alkoxycarbonyl, amino, alkylamino or dialkylamino), benzyl or saturated or unsaturated heterocyclyl having 5 or 6 members and 1-3 heteroatoms; or NR₁R₂ is a 5 or 6 membered heterocycle which may contain a further N, O or S heteroatom and may be alkylated; R₃ is H or alkyl and n is 2-4; and the alkyl moieties contain 1-4C.

Any suitable compositions or combinations of compositions including therapeutically effective amounts of cyclosporine component may be employed. The cyclosporine component is present in an amount effective to provide the desired therapeutic effect when the cyclosporine-containing composition is administered to a human or animal in accordance with the present invention. The cyclosporine component advantageously is present in the compositions in amounts ranging from about 0.01% to about 15% or about 20% or more by weight of the composition. In one embodiment, the cyclosporine component is present in an amount of about 0.02% to about 1% or about 5% or about 10% by weight of the composition. It is intended, however, that the choice of a particular amount of cyclosporine component is to be made in accordance with factors well known in the medicinal arts, including mode of administration, the size and condition of the human or animal and the type and severity of the condition to be treated.

The presently useful compositions may be liquids, suspensions, emulsions, semi-solids, gels, lotions, creams and the like. Those skilled in the art of pharmaceutical formulation are able to formulate suitable compositions including cyclosporine components in a suitable form, such as those forms noted herein, for example, including one or more pharmaceutically acceptable excipients, such as those conventionally used in similar compositions. Of course, care should be taken to use composition components which are compatible with the cyclosporine component being used and which do not unduly or significantly interfere with the administering step in which the composition is being used or with the human or animal being treated.

For example, cyclosporine components can be combined with carriers which form emulsions upon mixing with water. Such emulsions are described, for example, and without limitation, in Cavanak U.S. Pat. No. 4,388,307.

Carriers, for example, and without limitation, glyceride carriers, may assist in alleviating problems of physical instability such as precipitation of the cyclosporine component from solution, and may also enable higher blood plasma concentrations, if desired.

In a useful embodiment, the presently useful compositions include hydrophobic components. Any suitable hydrophobic component may be employed in the present invention. Advantageously, the cyclosporine component is solubilized in the hydrophobic component. In one embodiment, the hydrophobic component may be considered as comprising a discontinuous phase in the presently useful cyclosporine component-containing compositions, for example, oil-in-water emulsions.

The hydrophobic component may be present in an effective amount, for example, in an amount of about 0.1%-1.5%, 0.1%-10%, 1.0%-1.5% or 1.0%-10% by weight of the composition.

Preferably, the hydrophobic component comprises one or more oily materials. Examples of useful oil materials include, without limitation, vegetable oils, animal oils, mineral oils, synthetic oils and the like and mixtures thereof. In a very useful embodiment, the hydrophobic component comprises one or more higher fatty acid glycerides. Excellent results are obtained when the hydrophobic component comprises castor oil.

Other useful cyclosporine component-containing compositions comprise the cyclosporine component in admixture with an emulsifying amount of a fatty acid glyceride, such as castor oil and the like, and a surfactant, such as polysorbate 80. Such compositions are described in Ding et al U.S. Patent 5, 474, 979.

Also see Kaswan U.S. Patent 4,649,047 and Kaswan U.S. Patent 5, 411, 952.

In one embodiment, the presently useful compositions are self-emulsifying which, when exposed to an aqueous medium, form fine oil-in-water emulsions with little or no agitation. The property of self-emulsification permits such formulations to be administered in concentrated form, as for example in a hard gelatin or soft elastic capsules, with the expectation that a fine emulsion will be formed in the digestive tract. Additionally, emulsions may be prepared by combining a self-emulsifying pre-concentrate with an aqueous medium.

Previously disclosed self-emulsifying systems include those in which a cyclosporine component is combined with mixtures of (i) medium-chain triglycerides and nonionic surfactants, (ii) vegetable oils and partial glycerides, such as polyglycolized glycerides or medium-chain mono- and diglycerides, or (iii) vegetable oils and nonionic surfactants such as polysorbate 80 or PEG-25 glyceryl trioleate.

In certain self-emulsifying formulations, a "microemulsion preconcentrate" of a cyclosporine component is formed by combining the cyclosporine component with (I) a hydrophilic phase, (II) a lipophilic phase, and (III) a surfactant, as well as optional thickeners, antioxidants or other excipients. Examples of such compositions are disclosed in Hauer et al U.S. Patent 5,342,625.

In addition, suitable compositions may include cyclosporine components in combination with a hydrophilic solvent phase and one or more surfactants, but not containing lipophilic solvents. Such cyclosporine component-containing formulations may be stable, simple to prepare, and have good pharmacokinetic properties.

As used herein, the terms "binary system", "binary composition" and "binary system of excipients" denote those cyclosporine component-containing formulations and compositions which contain, in addition to the cyclosporine component, a combination of at least one hydrophilic solvent and at least one surfactant, but which lack a lipophilic solvent. Such compositions may be supplemented with additional adjuvants and still be considered "binary", so long as they do not include a lipophilic solvent phase.

To prepare such pharmaceutical compositions, a binary system is combined with a cyclosporine component. The hydrophilic phase may comprise one or more of the known pharmaceutically acceptable hydrophilic solvents or excipients that are capable of solubilizing the cyclosporine component. Suitable classes of hydrophilic compounds include, for example and without limitation, pharmaceutically acceptable alcohols including the polyethylene glycols.

Examples of hydrophilic phase components useful in the presently useful compositions include, but are not limited to, water, ethanol, benzyl alcohol, propylene glycol, low molecular weight polyethylene glycols having a molecular weight of up to about 1,000, glycol, dimethyl isosorbide and the like and mixtures thereof.

The hydrophilic phase, comprising one or more hydrophilic solvents, typically comprises about 10% to about 90% by weight of the pharmaceutical composition. The precise amount used will vary depending on the nature of the hydrophilic compound or compounds used, the amount of cyclosporine component present, the dosage form, the condition being treated and other factors known in the art. Preferably the hydrophilic phase comprises about 20% to about 80%, and more preferably about 30% to about 60%, by weight of the composition. Where non-aqueous hydrophilic components are used, water can be included in the formulation at levels varying from about 0.5% to about 10%, or preferably from about 1% to about 5%, based on total weight of the composition.

Any of the known pharmaceutically acceptable surfactants may be used, including nonionic, anionic, cationic, and combinations thereof. Nonionic surfactants are preferred, and especially those surfactants having a hydrophile/lipophile balance (HLB) of 10 or more. Alternatively, certain combinations of high- and low-HLB surfactants may be utilized; preferably, such mixed surfactants are used in ratio such that the aggregate surfactant HLB (when weighted according to proportions used) remains in excess of 10.

Examples of suitable surfactants include, but are not limited to, polyoxyethylene derivatives of natural or hydrogenated vegetable oils such as castor oil; polyoxyethylene-sorbitan fatty acid esters, such as mono-, di- and tri-lauryl, palmityl, stearyl and oleyl esters; alkyl/dialykyl sulfate, sulfonate or sulfosuccinate salts such as sodium lauryl sulfate and dioctyl sodium sulfosuccinate; polyoxyethylene fatty acid esters; phospholipids such as lecithins; transesterification products of natural vegetable oil triglycerides and polyalkylene polyols; sorbitan fatty acid esters; pentaerythritol fatty acid esters; polyoxyethylene glycol alkyl ethers and esters; and the like. The surfactants may be used alone or in combination.

Examples of specific surfactants which may be used include, without limitation, polyoxyethylene castor oil derivatives, such as polyoxyethylene glycerol triricinoleate polyoxyl 35 castor oil (CREMOPHOR® EL, available from BASF Corporation), and polyoxyl 40 hydrogenated castor oil (CREMOPHOR® RH40, available from BASF Corporation); mono-fatty acid esters of poloxyethylene (20) sorbitan, such as polyoxyethylene (20) sorbitan monooleate (TWEEN® 80), polyoxyethylene (20) sorbitan monostearate (TWEEN® 60), polyoxyethylene (20) sorbitan monopalmitate (TWEEN® 40), and polyoxyethylene (20) sorbitan monolaurate (TWEEN® 20) (all available from ICI Surfactants, Wilmington, Del.); polyoxyethylene glycol 200 monostearate (MYRJ® 52, available from Calgene Chemicals, Skokie, Ill.); polyglycerol esters with a HLB of 10 or greater, such as decablyceryl mono- and dioleate and the like; and mixtures thereof.

In some instances (as when the compositions are prepared as semi-solids), it may be advantageous to use at least one additional low-HLB surfactant along with one or more of the above high-HLB surfactant. Examples of low-HLB auxiliary surfactants which may be used include, but are not limited to, polyglycerol oleates (such as CAPROL® 10G40); lecithins; glyceryl monooleate or monolinoleate mixtures (such as MYVEROL® 18-99 or 18-92); propylene glycol laurate; and sorbitan oleates such as sorbitan monooleate (SPAN® 80), sorbitan trioleate (SPAN® 85), and sorbitan sesquioleate (SPAN® 20) (all available from ICI Surfactants, Wilmington, Del.). The surfactant phase may comprise about 10% to 90% by weight of the composition. Preferably the surfactant comprises about 20% to about 70% of the composition, and more preferably about 40% to about 60%, by weight.

If desired, the presently useful compositions may additionally comprise other pharmaceutically acceptable excipients, such as tonicity components, buffer components, polyelectrolyte components, thickeners, fillers, diluents, flavoring agents, coloring agents, antioxidants, preservatives, such as antibacterial or antifungal agents, acids and/or basis to adjust pH, and the like and mixtures thereof. Such additives, if present, may typically comprise about 0.01% to about 10% by weight of the composition. Such additives include those additives which are conventional and/or well known for use in similar pharmaceutical compositions. For example, suitable thickening agents include any of those known in the art, as for example pharmaceutically acceptable polymers and/or inorganic thickeners. Such agents include, but are not limited to, polyacrylate homo-and co- polymers; celluloses and cellulose derivatives; polyvinyl pyrrolidones; polyvinyl resins; silicates; and the like and mixtures thereof.

When desired, the cyclosporine-containing compositions may be prepared as semi-solid rather than liquid formulations by addition a greater proportion of appropriate thickening or solidifying agents. As noted above, such preparations may be particularly useful as fills for hard gelatin (as opposed to soft gelatin) capsules. Solidifiers suitable for the preparation of semi-solid compositions include, but are not limited to, polyethylene glycols having a molecular weight of more than about 1,000, such as PEG 1450 and PEG 3350; stearyl alcohol; and colloidal silicon dioxide (CAB-O-SIL® M-5, available from Cabot, Tuscola, Ill.). A semi-solid state may be often obtained by adding between about 8% or about 10% and about 15% or about 25% by weight solidifying agent. The actual amount of solidifying agent needed will depend on the physical characteristics of the other excipients which are present.

Except as otherwise noted elsewhere herein, the cyclosporine component-containing compositions may be administered topically and/or systemically, for example, by any of the methods known in the art. Such methods include, but are not limited to, systemic administration, for example, oral administration of a suspension formed by mixing a cyclosporine component-containing composition with an aqueous medium such as water, milk or juice; a cyclosporine component-containing composition placed in a soft elastic or hard gelatin capsule; parenteral administration including intravenous, intramuscular, intraperitoneal, intrasternal, subcutaneous and intraarticular injection or infusion of a cyclosporine component-containing composition; and/or topical administration methods, such as topical administration of ointments, drops, solutions, suspensions or emulsions including a cyclosporine component. Topical formulations, intended for topical administration to the affected tissue area or areas, may be prepared directly, or by combining a cyclosporine component-containing concentrate with a diluent, for example, an aqueous diluent. Such topical formulations may include additional excipients as necessary, for example, to modify consistency of the rate of absorption of the cyclosporine component.

In preparing the presently useful compositions, the components may be combined in any order with mixing or light agitation to ensure complete blending.

The cyclosporine component may be administered in a sufficient amount, and for a sufficient time, as required to provide the desired therapeutic effect. The specific therapeutically effective dosage level may be dependent on a number of factors including the specific condition to be treated, the severity of the condition, the activity of the particular cyclosporine component being employed, the specific cyclosporine component-containing composition employed, the time and method of administration, the duration of treatment, and other factors which are well known in the medical arts.

In one useful embodiment, the cyclosporine component is topically applied to an ocular surface of an eye in an oil-in-water emulsion, for example a cyclosporin A-containing emulsion sold by Allergan, Inc. under the trademark Restasis®, once, twice or three times daily for a period of time in a range of about 1 day or about 1 week to about 3 weeks or about 4 weeks or longer before the IOL inserting step, and for a period of time in a range of about 1 week or about 1 month to about 2 months or about 4 months or longer after the IOL inserting step.

The intraocular lens (IOL) inserting step may be conducted in any suitable manner effective to place the IOL in the eye to provide benefit, for example and without limitation, enhanced vision, to the patient. Many such inserting steps are well known in the art and are conventional. Therefore a detailed explanation or description of such IOL inserting step is not presented here.

In general, the IOL inserting step comprises passing the IOL, for example, in a folded or rolled configuration, through a small incision in the ocular surface, for example, the sclera, of the eye, into a desired location in the eye, for example and without limitation, in the anterior chamber of the eye, the posterior chamber of the eye or elsewhere in the eye. The incision in the eye may range from about 1 mm or about 2 mm to about 5 mm or about 7 mm or larger, and is often in a range of about 2 mm to about 4 mm. After the IOL inserting step, the surgical instrument or instruments are removed from the eye and the incision is left to heal. One or more sutures may be provided to facilitate healing of the incision. In one embodiment, the incision is left unsutured.

In certain embodiments, the inserting step is preceded by a lens extraction step in which the natural lens, which may be damaged or injured, such as having a cataract condition, is removed. Any of a number of conventional and well known lens extraction steps may be employed. The incision used to insert the IOL may be the same incision through which the natural lens is extracted.

The IOL that is inserted in the eye may be any suitable IOL, many of which are conventional and well known in the art. Such lenses often include an optic which may be structured to provide one or more vision corrections, such as a spherical correction, a cylinder correction and the like, and/or may be multifocal and/or accommodating. The IOLs may be foldable or rollable or deformable so as to include optics made of flexible polymeric materials, such as silicones, acrylates and the like, with haptics or fixation members, which facilitate maintaining the IOL in position in the eye, made of polymethylmethacrylate and the like materials. Specific examples of useful IOLs in accordance with the present invention include, without limitation, those sold by Alcon under the tradename Restor, those sold by Advanced Medical Optics under the tradename ReZoom, and those sold by Eyeonicx under the tradename Crystalens.

The following non-limiting examples illustrate certain aspects of the present invention.

### Example I

A series of five (5) human patients ranging in age from 60-75 years old were identified with cataracts in one of their eyes. It was determined to surgically remove the natural lens from each of such eyes and insert a conventional multifocal IOL in the posterior capsule of each of the eyes, in place of the removed natural lens.

At least two (2) weeks before surgery, each of the patients topically applied one or more drops of a cyclosporin A-containing emulsion, sold by Allergan, Inc. under the trademark Restasis®, twice a day to the eye to be subjected to surgery. This emulsion included 0.05% by weight cyclosporin A, 1.25% by weight castor oil, 1.0% by weight polysorbate 80 and water.

The natural lens of each of the eyes was removed using a conventional phacoemulsification procedure which included forming a 3 mm (millimeter) incision in the sclera of the eye. Using conventional surgical techniques, the multifocal IOL was placed into the vacated posterior capsule of each of the eyes. The IOL was folded or rolled and passed through the above-noted "incision" into the eye. Once the lens was properly placed in the eye, the incision, without suturing, was left to heal.

After the surgery, each of the patients topically applied one or more drops of the above-noted cycloporin A-containing emulsion to the eye twice a day for three (3) months.

Each of the patients was examined post-operatively, three (3) months after surgery, and measurements of best corrected visual acuity, both near and far were taken. In addition, evaluations were made of contrast sensitivity, with and without glare testing, and uncorrected and best corrected visual acuity.

The results of these measurements and evaluations were compared with comparable intraocular lens insertion surgeries on comparable patients that were conducted without treating with cyclosporin A. Such comparison showed that the five (5) patients who used cyclosporin A, as noted above, showed improvement in regularity of the topography of the ocular surface, improvement in contrast sensitivity, and reduced wavefront aberrations in the surgically treated eye relative to the similarly surgically treated eyes of the comparable patients who did not use cyclosporin A.

### EXAMPLE II

To evaluate the effect of topical cyclosporine A 0.05% (Restasis; Allergan, Inc.; Irvine, CA), on quality of vision in patients undergoing cataract surgery with a multifocal intraocular lens (IOL).

Methods: A multicenter, randomized, masked, prospective clinical trial to evaluate the effect of cyclosporine A 0.05% versus an artificial tear on the quality of vision in 40 eyes of 20 patients bilaterally implanted with the R eZoom^{®} IOL (AMO, Santa Ana, CA). Masked study medications were instilled 2 times a day for 1 month preoperatively and 2 months postoperatively in one eye and the second eye randomly received the artificial tear. UCVA, BCVA, mesopic and photopic contrast sensitivity under high-contrast and low-contrast illumination, and topography were measured unilaterally at 2 months following implantation in each eye.

Results: At 2 months following the second eye implantation, the eyes that received the cyclosporine A had significantly improved mesopic and photopic contrast sensitivity compared to eyes not receiving cyclosporine A and there was a trend towards improved UCVA and BCVA in the cyclosporine A treated eyes.

Conclusions: The preoperative and postoperative use of cyclosporine A 0.05% significantly improved visual outcomes in patients implanted with the ReZoom^{®} multifocal IOL.

## Claims

1. A cyclosporine component, for use in a method of mitigating one or more of the detrimental effects of the insertion of an intraocular lens in an eye by enhancing visual acuity, contrast sensitivity, healing of the ocular surface, quality of vision or reducing wavefront aberrations in the eye of a patient who has received an intraocular lens, the method comprising:
topically applying a cyclosporine component to an ocular surface of an eye of a patient; and
inserting an intraocular lens in the eye of the patient,
wherein the cyclosporine component is to be applied prior to and after the inserting step.

2. The cyclosporine component for use in a method according to claim 1, wherein the cyclosporine component is present in an emulsion to be topically applied to the ocular surface

3. The cyclosporine component for use in a method according to claim 1, wherein the cyclosporine component is present in an amount in a range of about 0.01% to about 1% by weight in a composition to be topically applied to the ocular surface.

4. The cyclosporine component for use in a method according to claim 1, wherein the cyclosporine component is selected from the group consisting of cyclosporines, cyclosporine derivatives, salts thereof and mixtures thereof.

5. The cyclosporine component for use in a method according to claim 1, wherein the cyclosporine component is selected from the group consisting of cyclosporin A, derivatives of cyclosporin A, salts thereof and mixtures thereof.

6. The cyclosporine component for use in a method according to claim 1, wherein the cyclosporine component comprises cyclosporin A.

## Patentansprüche

1. Cyclosporinkomponente zur Verwendung in einem Verfahren zur Abschwächung einer oder mehrerer der schädlichen Auswirkungen des Einsetzens einer Intraokularlinse in ein Auge durch Verbesserung der Sehschärfe, Kontrastempfindlichkeit, Heilung der Augenoberfläche, Sehqualität oder Verringerung der Wellenfrontaberration im Auge eines Patienten, der eine Intraokularlinse erhalten hat, wobei das Verfahren umfasst:
Aufbringen einer topischen Cyclosporinkomponente auf eine Augenoberfläche des Auges eines Patienten; und
Einsetzen einer Intraokularlinse in das Auge eines Patienten,
wobei die Cyclosporinkomponente vor und nach dem Einsetzungsschritt aufgebracht werden soll.

2. Cyclosporinkomponente zur Verwendung in einem Verfahren gemäss Anspruch 1, wobei die Cyclosporinkomponente in einer Emulsion vorliegt, die topisch auf die Augenoberfläche aufgebracht werden soll.

3. Cyclosporinkomponente zur Verwendung in einem Verfahren gemäss Anspruch 1, wobei die Cyclosporinkomponente in einer Menge im Bereich von etwa 0,01 bis etwa 1 Gew.% in einer Zusammensetzung, die topisch auf die Augenoberfläche aufgebracht werden soll, vorliegt.

4. Cyclosporinkomponente zur Verwendung in einem Verfahren gemäss Anspruch 1, wobei die Cyclosporinkomponente aus der Gruppe bestehend aus Cyclosporinen, Cyclosporinderivaten, Salzen davon und Mischungen davon ausgewählt ist.

5. Cyclosporinkomponente zur Verwendung in einem Verfahren gemäss Anspruch 1, wobei die Cyclosporinkomponente aus der Gruppe bestehend aus Cyclosporin A, Derivaten von Cyclosporin A, Salzen davon and Mischungen davon ausgewählt ist.

6. Cyclosporinkomponente zur Verwendung in einem Verfahren gemäss Anspruch 1, wobei die Cyclosporinkomponente Cyclosporin A umfasst.

## Revendications

1. Composant de type cyclosporine destiné à être utilisé dans un procédé d'atténuation d'un ou plusieurs des effets défavorables de l'insertion d'une lentille intraoculaire dans un oeil par augmentation de l'acuité visuelle, de la sensibilité aux contrastes, de la cicatrisation de la surface oculaire, de la qualité de la vision ou réduction des aberrations du front d'onde dans l'oeil d'un patient qui a reçu une lentille intraoculaire, le procédé comprenant :
l'application topique d'un composant de type cyclosporine à une surface oculaire d'un oeil d'un patient ; et
l'insertion d'une lentille intraoculaire dans l'oeil du patient,
où le composant de type cyclosporine est destiné à être appliqué avant et après l'étape d'insertion.

2. Composant de type cyclosporine destiné à être utilisé dans un procédé selon la revendication 1 où le composant de type cyclosporine est présent dans une émulsion destinée à être appliquée par voie topique à la surface oculaire.

3. Composant de type cyclosporine destiné à être utilisé dans un procédé selon la revendication 1 où le composant de type cyclosporine est présent en une quantité dans une plage d'environ 0,01 % à environ 1 % en poids dans une composition destinée à être appliquée par voie topique à la surface oculaire.

4. Composant de type cyclosporine destiné à être utilisé dans un procédé selon la revendication 1 où le composant de type cyclosporine est choisi dans le groupe consistant en les cyclosporines, les dérivés de cyclosporine, leurs sels et leurs mélanges.

5. Composant de type cyclosporine destiné à être utilisé dans un procédé selon la revendication 1 où le composant de type cyclosporine est choisi dans le groupe consistant en la cyclosporine A, les dérivés de cyclosporine A, leurs sels et leurs mélanges.

6. Composant de type cyclosporine destiné à être utilisé dans un procédé selon la revendication 1 où le composant de type cyclosporine comprend la cyclosporine A.
